# EUROPEAN PATENT APPLICATION

(11) **EP 4 813 421 A1**
(43) Date of publication of application: **30.09.2026**
(21) Application number: 25166524.6
(22) Date of filing: 27.03.2025
(51) Int. Cl.: A61M 5/20, A61M 5/24, A61M 5/32

(54) **LOADING MECHANISM FOR A DRUG DELIVERY DEVICE**

(71) Applicant: Ypsomed AG, 3401 Burgdorf (CH)
(72) Inventor: Wegner, Stephan, 4051 Basel (CH); Wüthrich, Roman, 3700 Spiez (CH); Bernhard, Mario, 3400 Burgdorf (CH); Shepherd, David, Edinburgh, EH12 7HW (GB); Fischbacher, Peter Alastair, Edinburgh, EH12 7HW (GB); McLusky, James Donald, Edinburgh, EH6 4QN (GB)

(57) **Abstract**

The present invention relates to a loading mechanism for a drug delivery device for dispensing a liquid drug. The loading mechanism comprises a drive unit and a syringe insertable into the drive unit. The drive unit comprises a drive unit housing defining a longitudinal axis, a plunger movable inside the drive unit housing in a dispensing direction to dispense the liquid drug and a mechanical drive, preferably a compression spring, arranged inside the drive unit housing for moving, when loaded, the plunger in the dispensing direction. The drive unit housing comprises an insertion opening to insert and remove the syringe. The drive unit further comprises a puller that is movable in the proximal direction to pull the plunger in the proximal direction to load the mechanical drive. According to the invention, the puller is adapted to block the insertion opening before moving in the proximal direction and to open the insertion opening by moving in the proximal direction.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a drug delivery device comprising a preferably reusable drive unit and a syringe, preferably a safety syringe, that can be inserted into the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from the housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed of in the regular waste but must be subjected to special disposal or to recycling, which is an additional burden to the patient. Further, disposing of a battery, motor and/or electronics after a single use is a waste of resources and increases the costs of the therapy. Disposable autoinjectors comprising a mechanical drive, like a compression spring, are easier to dispose of, however it is also a waste of resources to dispose of the compression spring after a single use, which also increases the costs of the therapy.

To account for the need to handle and dispose of the autoinjector parts differently, semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection. A big problem for semi-reusable mechanical autoinjectors is that the compression spring has to be recompressed or re-loaded after each injection, which can require a high force and accuracy, which can be challenging for the user, especially for older patients. A related problem is that users might not remember if they have already re-loaded the injection spring. However, it can be dangerous if the user tries to insert the syringe unit when the spring is not loaded, due to the spring repulsing the syringe unit when the spring is only partly re-loaded. Another disadvantage is that some semi-reusable autoinjectors use syringe units with multiple components, so that there is still a waste of material when after each injection the syringe unit is discarded.

US 2006/069354 A1 discloses a semi-reusable autoinjector with a reusable drive unit comprising a drive spring and a syringe that can be inserted into the drive unit. To re-load the drive spring, the drive unit comprises a piston retraction yoke that a user can pull in a proximal direction away from the injection side of the drive unit. After the drive spring is loaded, the syringe can be laterally inserted into the drive unit through a hinged clamshell door. For the injection, the syringe is then pushed towards the desired injection site by the plunger so that the needle is inserted into the skin of the user and consequently the medicament is dispensed by further movement of the plunger. US 2011/0172602 A1 discloses a similar injection device.

These known semi-reusable autoinjectors are sometimes difficult to assemble and there is a relatively high risk that the user accidentally touches the needle because the syringe is not received in a syringe unit and the needle is therefore not covered after the injection. Another general problem is that it is dangerous for the user and can lead to injuries of the user if the user tries to insert the syringe before the retraction yoke is pulled back.

US 2013/138049 A1 discloses an autoinjector with a reusable drive unit comprising a drive spring and a syringe insertable in the drive unit. The syringe has a needle retraction mechanism to protect the needle after the injection is completed. To protect the needle before the injection, the syringe is located inside the drive unit in an initial state and is automatically moved out of the drive unit when the injection is triggered. To insert a new syringe into the drive unit, the drive unit housing can be opened around a lateral hinge. Upon opening the drive unit, the drive spring is reloaded by a lever arrangement. This device has the disadvantage that additional components and features are necessary to provide syringe movement and for the lever arrangement making the autoinjector more complex, more expensive and less fail-safe.

### DESCRIPTION OF THE INVENTION

It is an objective of the present invention to provide a drug delivery device with a mechanical drive that allows a user to load the drug delivery device by tensioning the mechanical drive and to insert the disposable syringe, in particular a safety syringe, before the injection and to remove the syringe after the injection in an easy and safe way.

This objective is achieved by the features of the independent claims. Preferred embodiments are evident from the dependent claims.

The invention relates to a loading mechanism for a drug delivery device for dispensing a liquid drug.

The loading mechanism comprises a drive unit and a syringe insertable into the drive unit. With the loading mechanism, a mechanical drive of the drive unit can be loaded, and the syringe can be inserted into the drive unit before each injection. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable syringe. After each injection the syringe is disposed and replaced by a new syringe. The syringe may preferably be a safety syringe with a needle retraction mechanism or an integrated needle cover.

The drive unit of the loading mechanism comprises a drive unit housing defining a longitudinal axis and a plunger movable inside the drive unit housing in a dispensing direction to dispense the liquid drug. The plunger can also be referred to as piston rod or drive element. The longitudinal axis defines a longitudinal direction of the drive unit. The longitudinal axis of the drive unit is also considered as the longitudinal axis of the drug delivery device. The dispensing direction can preferably be the longitudinal direction and more preferably a distal direction. The drive unit further comprises a mechanical drive arranged inside the drive unit housing for moving, when loaded with the loading mechanism, the plunger in the dispensing direction. Preferably, the mechanical drive is a compression spring. For loading, the mechanical drive is preferably compressed in a proximal direction, hence a distal end of the mechanical drive is moved in the proximal direction while the proximal end of the mechanical drive is fixed. The mechanical drive can be one compression spring that is arranged proximally to the plunger; hence the plunger can be arranged between the syringe and the compression spring. Alternatively, the mechanical drive can comprise one or preferably two compressions springs that is/are arranged parallel to the syringe, with other words next to or adjacent the syringe. This enables a shorter longitudinal dimension of the drive unit and hence the drug delivery device.

The drive unit housing comprises an insertion opening to insert the syringe into the drive unit and to remove the syringe from the drive unit after the injection is finished. Preferably the syringe is inserted into the drive unit housing by a user prior to an injection to assemble the drug delivery device and to prepare the drug delivery device for the injection. Hence, the inserting of the syringe into the drive unit housing does not take place during assembly of the drug delivery device or any other manufacturing step by the manufacturer, before the syringe and the drive unit are delivered to the user. Preferably, the syringe is fixed or non-movably arranged inside the drive unit housing. That means that during an injection the syringe and hence the needle does not move relative to the drive unit housing so that the user must insert or penetrate the injection needle into the injection site by a manual insertion force. Preferably, the injection can be triggered by moving a needle cover of the drive unit or the syringe or a trigger element of the drive unit in the proximal direction to release the plunger from a locked position. This can for example be achieved by pressing the drug delivery device and thereby the needle cover or the trigger element against the skin at a desired injection site.

The drive unit of the loading mechanism further comprises a puller that is movable in the proximal direction to pull or move the plunger in the proximal direction to load the mechanical drive. A distal portion of the puller can be telescopically received in the drive unit housing, so that when the puller is moved in the proximal direction, the puller moves out of the drive unit housing and protrudes further from the proximal end of the drive unit housing. The puller can for example be a pull handle or a pull sleeve. The puller can for example comprise a ring feature and/or ergonomically formed recesses and/or protrusions on a proximal end of the puller to make it easier for the user to grab and move the puller and to exert the force needed to load the mechanical drive. The drive unit can comprise a puller release button that a user has to press before the puller can be moved in the proximal direction. This makes sure that the user does not accidentally move the puller in the proximal direction.

According to the invention, the puller is adapted to block the insertion opening before moving in the proximal direction and to open the insertion opening by moving in the proximal direction such that the syringe is insertable into the drive unit housing or removable from the drive unit housing through the insertion opening. Once inserted, the syringe can be held in place by clips or other fixing means. With the loading mechanism according to the invention, the reservoir unit can only be inserted after the puller is pulled in the proximal direction which means that the mechanical drive is loaded. Before the puller is moved in the proximal direction, the insertion opening is blocked so that the syringe cannot be inserted. This means that with the inventive loading mechanism, in a first step the mechanical drive is loaded and in a second step the syringe is inserted into the drive unit housing. The loading of the mechanical drive can take place when the puller is moved to be able to insert a new syringe or when the puller is moved to be able to remove the used syringe after the injection.

An advantage of the loading mechanism according to the invention is that the puller prevents the user from inserting the syringe before the mechanical drive is loaded in a way that is easy to understand and easy to perform. Therefore, the loading mechanism provides a safe and easy way to prepare the medicament delivery device for an injection. A further advantage is that the puller can open the insertion opening and load the mechanical drive. Hence no other components and user steps are necessary which makes the design and functionality of the drug delivery device particularly easy. Lastly, the loading mechanism allows the use of a syringe, preferably a safety syringe, as a reservoir so that syringe is the only disposable component of the drug delivery device which significantly reduces waste of material and reduces the manufacturing effort as no final assembly is necessary to use the syringe as the medicament reservoir.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In a preferred embodiment, the insertion opening is located laterally on the drive unit housing so that the syringe is laterally insertable. Preferably, laterally means that the syringe is inserted in a direction perpendicular to the longitudinal axis defined by the drive unit. This makes it easy to open and close the insertion opening by moving the puller in the proximal or distal direction. It is also comfortable for the user to insert the syringe, because the user does not have to guide the syringe through a narrow opening on the distal or proximal end of the drive unit but can easily place the syringe inside the drive unit before closing the drive unit by moving the puller in the distal direction.

In a preferred embodiment, the puller comprises a first projection and the plunger comprises a second projection that abut, when the puller is moved in the proximal direction to pull the plunger in the proximal direction. Due to the abutment, the plunger moves in the proximal direction together with the puller, which loads the mechanical drive. This provides a design that is reliable and easy to manufacture and assemble. When the puller is moved back in the distal direction to close the medicament delivery device, the first and second projection no longer abut, so that only the puller is moved back, but the plunger does not move together with the puller. Hence, the projections make it easy to move the plunger together with the puller only in the proximal direction but not in the distal direction.

In a preferred embodiment, the puller is adapted to remove a needle shield, preferably a rigid needle shield, of the syringe, when the puller is moved in the distal direction to close the drug delivery device. When the drug delivery device is closed, the puller at least partially covers or hides the syringe plunger and syringe flange. This preferred embodiment makes it easy to remove the needle shield because it does not require an additional user step and at the same time ensures that the user does not accidentally touch the needle while removing the needle shield. To remove the needle shield, the puller can directly contact or abut the needle shield to move the needle shield in the distal direction. Alternatively, the drive unit can comprise a needle shield pusher arranged between the puller and the needle shield to translate the movement of the puller to the needle shield.

In an alternative preferred embodiment, the drive unit comprises a push button that is adapted to remove a needle shield of the syringe. The push button can directly contact the needle shield, or it can be connected to the needle shield with a lever arrangement. This also makes it easy for the user to remove the needle shield and to understand the user steps that are necessary to prepare the drug delivery device for an injection. It also prevents the user from accidentally touching the needle, as the user does not touch the needle shield with the fingers or the hand.

In another alternative preferred embodiment, the drive unit comprises a pivoting lever that automatically removes a needle shield of the syringe, when the syringe is inserted into the drive unit. The pivoting lever can catch or contact the distal end of the needle shield at the start of the insertion movement of the syringe and then push the needle shield off in the distal direction while the syringe is further inserted into the drive unit. In this alternative embodiment, no additional user step is necessary to remove the needle shield, which makes the drug delivery device easy to understand and handle.

**In** all three preferred embodiments for needle shield removal described above, the needle shield can be pushed in the distal direction for a distance of at least 5 mm. This movement breaks the seal of the syringe and moves the needle shield to a position where it will fall off the syringe due to gravity or can be pulled off the syringe by the user easily without requiring a high force.

**In** a preferred embodiment, the drive unit comprises a needle cover that is biased in the distal direction by a torsion spring. This makes it possible to change and/or reduce the dimensions of the drive unit housing compared to drive units that use a spiral spring to bias the needle cover in the distal direction. This embodiment can also be used for drug delivery devices with a trigger element instead of a needle cover, for example when the syringe is a safety syringe with an integrated needle shield. In these drug delivery devices, the trigger element can be biased in the distal direction by the torsion spring.

In a preferred embodiment, the syringe is inserted laterally through a needle cover of the drive unit, wherein the needle cover is reversibly moved to an open position by the lateral insertion of the syringe to enable the insertion of the syringe. With other words, the syringe opens the needle cover so that the syringe can be pushed through the needle cover and after the syringe is inserted, the needle cover returns to a closed state to circumferentially cover the needle and prevent accidental contact with the needle. Preferably, the needle shield of the syringe can push through the needle cover to temporarily open the needle cover. This makes it easy for the user to insert the syringe and at the same time provide secure protection of the needle. This preferred embodiment can also be provided for other drug delivery devices and is therefore not necessarily dependent on the other aspects or preferred embodiments described herein.

Preferably, the needle cover comprises a section movable around a hinge, or a section made of deformable material. It is also possible to provide two or more hinges or two or more deformable sections. This enables the opening of the needle cover to insert the syringe in an easy and reliable way.

A second aspect of the present invention relates to a loading mechanism for a drug delivery device for dispensing a liquid drug.

The loading mechanism comprises a drive unit and a syringe insertable into the drive unit. With the loading mechanism, a mechanical drive of the drive unit can be loaded, and the syringe can be inserted into the drive unit before each injection. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable syringe. After each injection the syringe is disposed and replaced by a new syringe. The syringe may preferably be a safety syringe with a needle retraction mechanism or an integrated needle cover.

The drive unit of the loading mechanism comprises a drive unit housing defining a longitudinal axis and a plunger movable inside the drive unit housing in a dispensing direction to dispense the liquid drug. The plunger can also be referred to as piston rod or drive element. The longitudinal axis defines a longitudinal direction of the drive unit. The longitudinal axis of the drive unit is also considered as the longitudinal axis of the drug delivery device. The dispensing direction can preferably be the longitudinal direction and more preferably a distal direction. The drive unit further comprises a mechanical drive arranged inside the drive unit housing for moving, when loaded with the loading mechanism, the plunger in the dispensing direction. Preferably, the mechanical drive is a compression spring. For loading, the mechanical drive is preferably compressed in a proximal direction, hence a distal end of the mechanical drive is moved in the proximal direction while the proximal end of the mechanical drive is fixed.

The drive unit housing comprises a proximal housing part and a distal housing part connected by a hinge so that the drive unit can be opened by moving the housing parts around the hinge. With other words, the drive unit housing can be opened by tilting or bending the housing parts relative to each other. When the drive unit is open, the syringe can be inserted into the drive unit housing, preferably into the distal housing part. Preferably the syringe is inserted into the drive unit housing by a user prior to an injection to assemble the drug delivery device and to prepare the drug delivery device for the injection. Hence, the inserting of the syringe into the drive unit housing does not take place during assembly of the drug delivery device or any other manufacturing step by the manufacturer, before the syringe and the drive unit are delivered to the user. Preferably, the syringe is fixed or non-movably arranged inside the drive unit housing. That means that during an injection the syringe and hence the needle does not move relative to the drive unit housing so that the user must insert or penetrate the injection needle into the injection site by a manual insertion force. Preferably, the injection can be triggered by moving a needle cover of the drive unit or the syringe or a trigger element of the drive unit in the proximal direction to release the plunger from a locked position. This can for example be achieved by pressing the drug delivery device and thereby the needle cover or the trigger element against the skin at a desired injection site. Once inserted, the syringe can be held in place by clips or other fixing means.

According to the second aspect of the invention, the proximal housing part of the drive unit housing comprises a turning knob to load the mechanical drive by a rotational movement of the turning knob around the longitudinal axis. With other words, the user must twist the turning knob to load the mechanical drive. This makes it possible to separate the user steps of inserting the syringe and loading the mechanical drive so that the user does not have to exert a high force while inserting the syringe. Additionally, due to the turning movement and the resulting force translation, it is easier for the user to load the mechanical drive, because the force needed can be reduced in comparison with a longitudinal loading movement. To further facilitate the loading of the mechanical drive, the drive unit can comprise a ratchet to aid the turning of the turning knob. Preferably, the drug delivery device comprises an indicator, for example a visible indicator connected to the plunger, that shows the user whether the mechanical drive is loaded or not respectively whether the mechanical drive is fully loaded.

An advantage of the loading mechanism according to the second aspect, is that it provides a safe and easy way to prepare the medicament delivery device for an injection. Additionally, the loading mechanism allows using a syringe, preferably a safety syringe, as reservoir so that syringe is the only disposable component of the drug delivery device which significantly reduces waste of material and reduces the manufacturing effort as no final assembly is necessary to use the syringe as the medicament reservoir.

In a preferred embodiment, the syringe is insertable into the distal housing part in a longitudinal direction from a proximal opening of the distal housing part. Hence, the syringe is insertable by a linear movement. Preferably, a detent can hold the drive unit housing respectively the hinge in an open position, so that it is easier for the user to insert the syringe. After the syringe is inserted, the user can close the drive unit housing. Preferably, the drive unit housing comprises a releasable clip that holds the drive unit housing in the closed position. Preferably, the syringe can also be removed from the drive unit housing by a linear movement in the longitudinal direction. This makes it particularly easy for the user to understand how the syringe is inserted or removed and to actually insert or remove the syringe.

In a preferred embodiment, the plunger comprises a lead screw and the proximal housing part comprises a thread compatible to the lead screw of the plunger, preferably on an inner surface of the proximal housing part, to translate or transform the rotational movement of the turning knob to a proximal movement of the plunger. In this embodiment, the plunger can preferably also perform a rotational movement to dispense the medicament. The lead screw provides a good force transmission, so that the mechanical drive can be reduced in size which allows to reduce the overall dimensions of the drug delivery device.

In a preferred embodiment, the drive unit comprises an opening button, preferably a push button, to open the drive unit and an injection trigger element movable between an extended position in which the push button can be pressed and a retracted position in which it releases the plunger and blocks the push button. This means that the device cannot be accidentally opened during the injection, when the trigger element is in the retracted position. The trigger element can for example be a needle cover of the drive unit. Alternatively, the trigger element can be a separate component, particularly when the syringe is a safety syringe with an integrated needle cover.

In a preferred embodiment, the syringe is a safety syringe with a needle retraction mechanism or a safety syringe with an integrated needle cover. This provides a particularly safe drug delivery device, especially during the syringe is removed after the injection. Both options prevent the user from accidentally touching the needle in a very secure way. With the needle retraction mechanism, the needle is retracted into the syringe at the end of the injection, for example into the stopper of the syringe. With the integrated needle shield, the needle shield is automatically moved in the distal direction to cover the needle after the injection is completed. This makes it possible to omit an additional needle cover of the drive unit or other safety measures, such as a safety cap, so that the drug delivery device has a very easy design. In case the drive unit comprises a needle cover, the needle cover does not have to provide the needle safety after the injection, for example if the drug delivery device falls to the ground and can therefore have a simpler and/or smaller design.

The invention also relates to a drug delivery device comprising an inventive loading mechanism according to the first or second aspect described above. Preferably, the drive unit housing, the plunger, the puller, the needle cover, the turning knob and/or the trigger element are injection-molded thermoplastic plastic parts. The injection-molded parts allow for a cost-effective production of the drug delivery device parts in particular in case of a high-volume production.

### BRIEF DESCRIPTION OF THE DRAWINGS

The subject matter of the invention will be explained in more detail in the following text with reference to preferred example embodiments which are illustrated in the attached drawings, in which depict:
- Fig. 1: a first example embodiment of an inventive drug delivery device comprising a drive unit and a syringe;
- Fig. 2: the drive unit of the drug delivery device of fig. 1 in an exploded view;
- Fig. 3: a schematic presentation of a loading and injection process of the drug delivery device of fig. 1 and 2;
- Fig. 4: the drug delivery device of fig. 1 to 3 before the syringe is inserted into the drive unit;
- Fig. 5: a first example embodiment of a needle cover of a drug delivery device according to the invention;
- Fig. 6: a second example embodiment of a needle cover of a drug delivery device according to the invention;
- Fig. 7: a third example embodiment of a needle cover of a drug delivery device according to the invention;
- Fig. 8: an example embodiment of an ejection button of a drug delivery device according to the invention to remove a needle shield of the syringe;
- Fig. 9: an example embodiment of a pivoting lever of a drug delivery device according to the invention to remove a needle shield of the syringe;
- Fig. 10: a second example embodiment of an inventive drug delivery device comprising a drive unit and a syringe;
- Fig. 11: the drive unit of the drug delivery device of fig. 10 in an exploded view;
- Fig. 12: a schematic presentation of a loading and injection process of the drug delivery device of fig. 10 and 11;
- Fig. 13: the drug delivery device of fig. 10 to 12 before and during the injection;
- Fig. 14: the drug delivery device of fig. 10 to 13 after the injection;
- Fig. 15: a third example embodiment of an inventive drug delivery device comprising a drive unit and a syringe;
- Fig. 16: the drive unit of the drug delivery device of fig. 15 in an exploded view;
- Fig. 17: a schematic presentation of a loading and injection process of the drug delivery device of fig. 15 and 16;
- Fig. 18: an example embodiment of a needle cover spring of a drug delivery device according to the invention in a top view; and
- Fig. 19: the embodiment of fig. 18 in a perspective view.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located. The term "proximal" refers to the opposite side or rear end of the device.

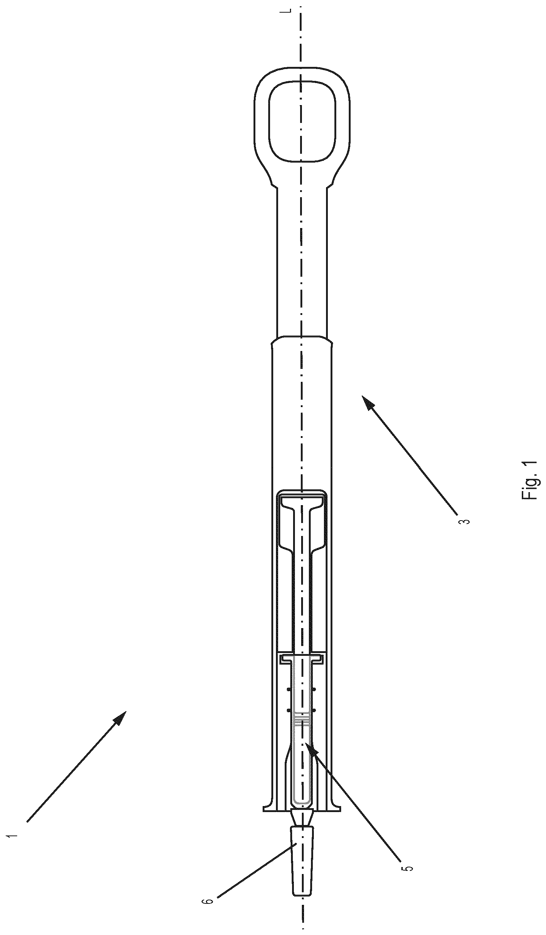

Fig. 1 shows an example embodiment of a drug delivery device 1 of the present disclosure in form of a semi-reusable autoinjector. Autoinjector 1 comprises a reusable drive unit 3 and a syringe 5 insertable into drive unit 3. The syringe 5 is laterally insertable into drive unit 3 from one side of the drive unit 3 by a linear movement. This linear movement is perpendicular to a longitudinal direction along a longitudinal axis L defined by the drive unit 3. In this embodiment, the syringe is a safety syringe with a needle retraction mechanism that retracts the needle into the syringe at the end of the injection. However, the drug delivery device 1 of fig. 1 could also be used for other kinds of safety syringes or for normal prefilled syringes. The drive unit 3 of this example embodiment has a length of 241 mm in the longitudinal direction and a rectangular cross section with outer dimensions of 34 mm and 26 mm.

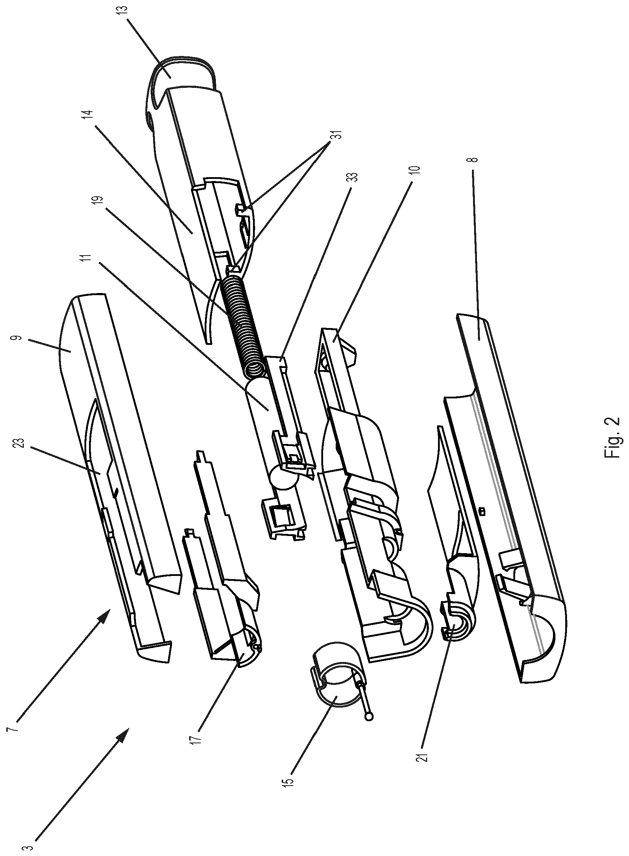

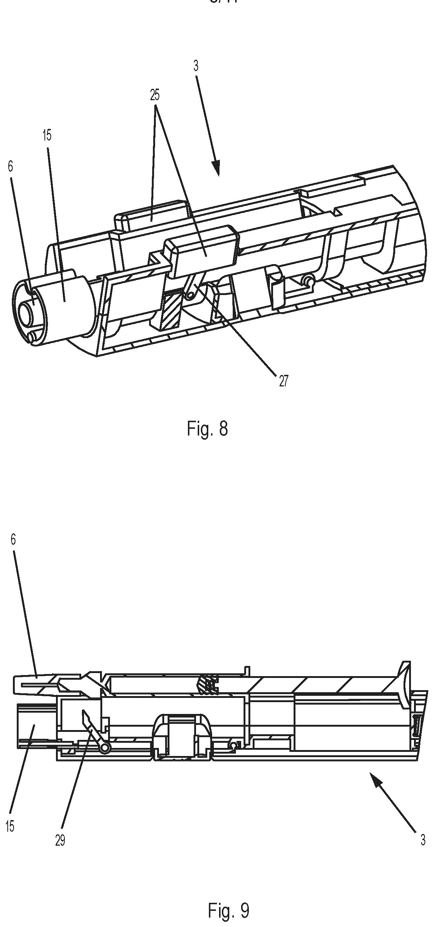

Fig. 2 shows the drive unit 3 of the autoinjector 1 in an exploded view. The main components of the drive unit 3 are a drive unit housing 7 comprising a lower housing part 8, an upper housing part 9 and a syringe holder 10; a plunger 11; a mechanical drive 19 in form of a compression spring; and a puller 13 in form of a pull handle. Drive unit 3 also comprises a needle cover 15, a trigger element 17 to trigger the injection when the autoinjector 1 is pressed against the skin at a desired injection site, and a pusher element 21 to remove a needle shield 6 of the syringe 5 (see fig. 1). Instead of the pusher element, the drive unit can comprise other features to remove the needle shield of the syringe that will be explained below with reference to fig. 8 and 9. The upper housing part 8 comprises an insertion opening 23 in a sidewall of the upper housing part 8, with other words the insertion opening 23 is arranged laterally on the drive unit housing 7. Through the insertion opening 23, the syringe 5 can be laterally inserted and removed. The syringe 5 is then received in a recess in the syringe holder 10 and held in place by clips that for example grab the syringe flange to fix the syringe 5 inside the syringe holder 10.

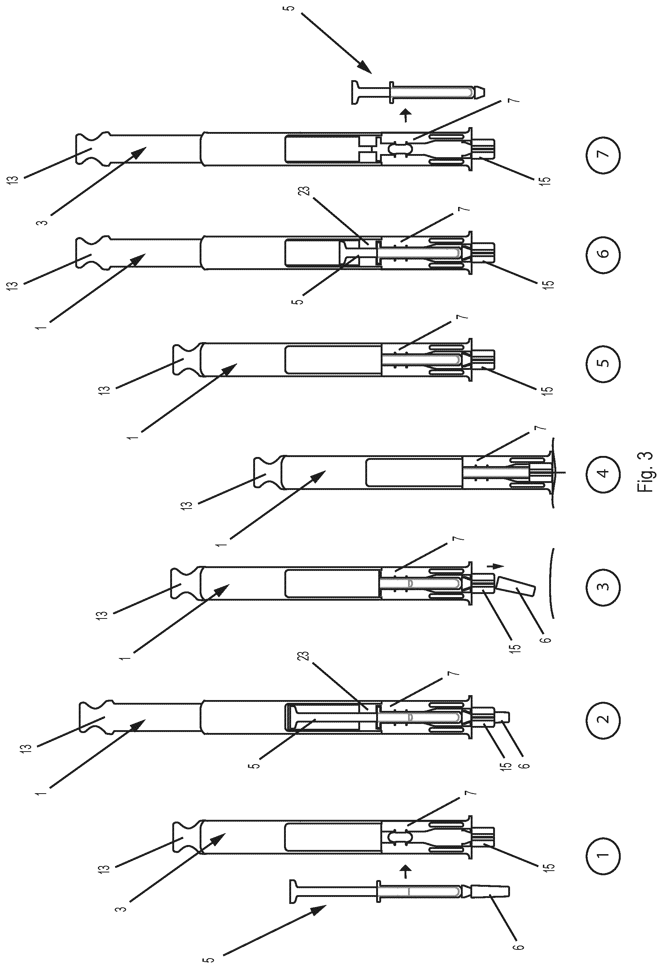

Fig. 3 is a schematic presentation of the loading and injection process of the drug delivery device 1 of fig. 1 and 2.

In a first step, the drive unit 3 is closed, hence the puller 13 is in its most distal position, and a new syringe 5 is separate from the drive unit 3. In the distal position, the puller 13 blocks the insertion opening 23 of the drive unit housing 7. To block the insertion opening 23, the puller 13 comprises a plate -like or flat protrusion 14 on its distal end (see fig. 2).

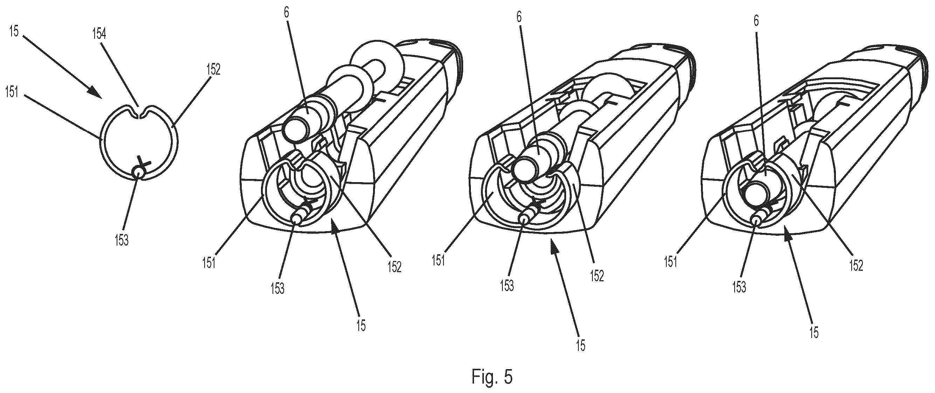

In the second step, the insertion opening 23 is opened by moving the puller 13 in the proximal direction and the syringe 5 is inserted into the drive unit 3 through the insertion opening 23. The mechanical drive (not shown in fig. 3) can already be loaded when the drive unit 3 is closed in the first step due to a previous proximal movement of the puller 13, for example to remove a previously used syringe, or it can be loaded by moving the puller 13 in the proximal direction in the second step. In both cases the mechanical drive is fully loaded after the second step so that the syringe 5 can easily be inserted into drive unit 3. To insert syringe 5 into drive unit 3, the syringe 5, specifically the needle shield 6 of the syringe 5, is laterally inserted through the needle cover 15 with other words pushed through the needle cover 15 of the drive unit 3. This means that the needle cover 15 is reversibly moved to an open position by the lateral insertion of the syringe to enable the insertion of the syringe. After the syringe is inserted, the needle cover 15 moves back to a closed position to fully cover the needle. Possible designs of the needle cover to enable this kind of syringe insertion are explained in detail later with reference to fig. 5 to 7.

In a third step, the puller 13 is moved back in the distal direction to close the drive unit 3 respectively block the insertion opening 23 and to secure the syringe 5 inside the drive unit 3. This return stroke of the puller 13 also removes the needle shield 6 from syringe 5 due to the pusher element (not shown in fig. 3) arranged between the puller 13 and the needle shield 6 that moves in the distal direction together with the puller 13 and thereby pushes the needle shield 6 in the distal direction. By this the needle shield 6 falls off or can easily be removed by the user. As can be seen in the third image of fig. 3, the puller 13 then covers or hides the syringe plunger and syringe flange.

In a fourth step, the user presses the autoinjector 1 against the skin at a desired injection site with the needle cover 15, which is the most distal part of the autoinjector 1, until the injection is complete, or until the user wants to end the injection due to other reasons. The skin contact retracts the needle cover 15 into the drive unit housing 7 and is thereby guided by the drive unit housing 107. This releases the plunger (not shown in fig. 3) by pushing the trigger element (not shown in fig. 3) in the proximal direction which releases the plunger. The plunger then pushes a stopper of the syringe in the distal direction to dispense the medicament from the syringe. After the injection is complete, in a fifth step, the autoinjector 1 is lifted from the skin, which releases the needle cover 15 to move back to its distal position to cover the needle of the syringe 5 and prevent the user from accidentally touching the needle during the following user steps.

To remove the empty syringe 5, in a sixth step, the puller 13 can be moved in the proximal direction to open the drive unit 3 and to reveal the syringe 5 so that the empty syringe 5 can be removed in a last user step. To remove the syringe 5, the user can for example push the syringe 5 out of the drive unit housing 7 by pressing a finger through a small opening in the lower housing part 8 opposite the insertion opening 23 in the upper housing part 9 of the drive unit housing 7. Alternatively, the drive unit 3 can comprise a button that the user can push to activate a lever to eject the syringe 5. The proximal movement of the puller 13 can preferably already load the mechanical drive for the next injection with a new syringe 5.

Referring to fig. 2, the loading of the mechanical drive 19 when the puller 13 is moved in the proximal direction is explained. When the puller 13 is moved in the proximal direction, the puller 13 pulls the plunger 11 in the proximal direction, which compresses the mechanical drive 19 arranged between the plunger 11 and a proximal inner end of drive unit housing 7. To pull the plunger 11, the puller 13 comprises two first projections 31 and the plunger 11 comprises two second projections 33, one of which is visible in fig. 2. The puller projections 31 are arranged distal from the plunger projections 33 so that they abut each other, when the puller 13 is moved in the proximal direction and the movement of the puller 13 is translated to a movement of the plunger 11. **In** some embodiments, the puller projections 31 and the plunger projections 33 can be arranged at a distance to each other when the puller 13 is in the most distal position so that they abut only after the puller 13 is moved a certain distance in the proximal direction. With other words, the movement of the puller 13 can be longer than the plunger movement to load the mechanical drive 19.

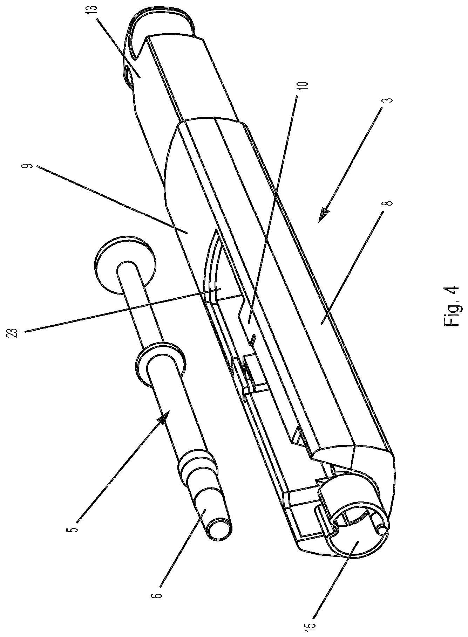

Fig. 4 shows a perspective view of the drive unit 3 of fig. 1 to 3 and the syringe 5 before the syringe 5 is inserted into the drive unit 3. Fig. 4 illustrates the recess in the syringe holder 10 and the insertion opening 23 in the upper housing part 9 of the drive unit housing 7.

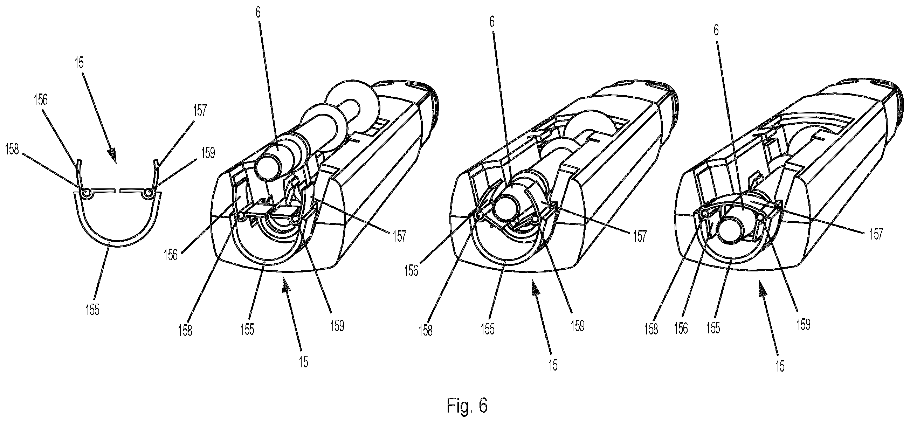

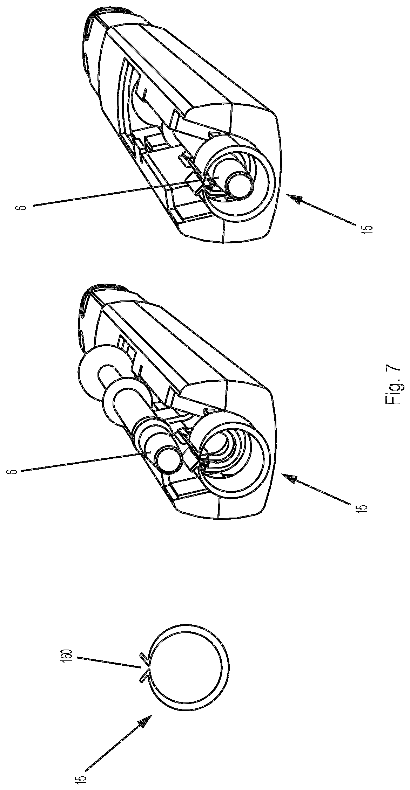

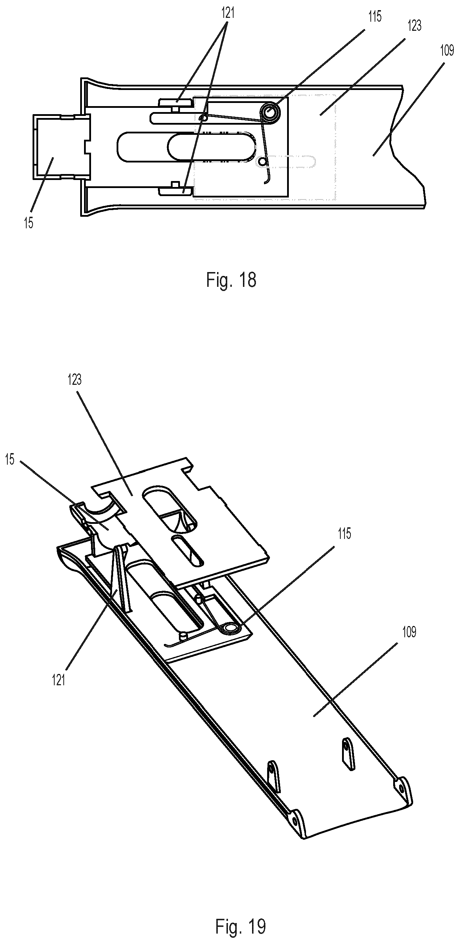

Fig. 5 to 7 show three different embodiments of the needle cover 15, that enable the needle shield 6 of syringe 5 to push through the needle cover 15, when the syringe 5 is inserted into the drive unit 3. **In** Fig. 7, the needle cover 15 comprises two half sleeves 151, 152 that are connected by a hinge 153. Opposite the hinge 153, the half sleeves each comprises a bent end that together form an entrance 154 for the needle shield 6 of the syringe 5. When the needle shield 6 pushes against the needle cover 15, the half sleeves 151, 152 move around the hinge 153, such that the entrance 154 becomes big enough to allow the needle shield 6 to pass through. When the needle shield 6 is fully inside the needle cover 15, the half sleeves 151, 152 move back to their original closed position so that the needle cover 15 fully encloses the needle shield 6 and the needle. **In** Fig. 8, the needle cover 15 comprises a bottom half 155 and two door portions 156, 157 that are each connected to the bottom half 155 by a hinge 158, 159. As shown in fig. 6, in this embodiment, the door portions are L-shaped. **In** a first closed position, a first arm of each of the door portions 156, 157 together with the bottom half 155 together form an almost closed sleeve. When the syringe 5 is inserted, the needle shield 6 pushes against the first arms of the door portions 156, 157 so that the door portions 156, 157 are rotated around the hinges 158, 159, such that the distance between the door portions 156, 157 becomes big enough to allow the needle shield 6 to pass through. When the needle shield 6 is fully inside the needle cover 15, second arms of the door portions 156, 157 have moved to the position where the first arms were in the initial position of the door portions 156, 157, so that the second arms then close the needle cover 15 so that the needle is fully covered by the needle cover 15. **In** Fig. 19, the needle cover 15 is made of an elastic material and has an opening 160. When the needle shield 6 pushes against the needle cover 15, the elastic material of the needle cover elastically deforms such that the entrance 160 becomes big enough to allow the needle shield 6 to pass through. When the needle shield 6 is fully inside the needle cover 15, the elastic material of the needle cover 15 returns to the initial position, so that the needle cover 15 fully encloses the needle.

Fig, 8 and 9 show two alternative embodiments to remove the needle shield 6 of the syringe 5 before the injection, that can be implemented instead of the pusher element shown in fig. 2. **In** Fig. 8, the drive unit 3 comprises two push buttons 25 that a user can press to remove the needle shield 6. The push buttons 25 are connected to the needle shield 6 over a lever arrangement 27, so that the movement of the push buttons 25 in a radial direction is translated into a distal movement of the needle shield 6. In other embodiments, the drive unit could also comprise only one push button that the user presses to remove the needle shield. In Fig. 9, the drive unit comprises a pivoting lever 29 that catches a proximal end surface of the needle shield 6 when the syringe 5 is inserted and translates the further movement of the syringe 5 in the radial direction during the insertion of the syringe 5 into a distal movement of the needle shield 6 so that the needle shield 6 is automatically removed when the syringe 5 is inserted without a separate user step.

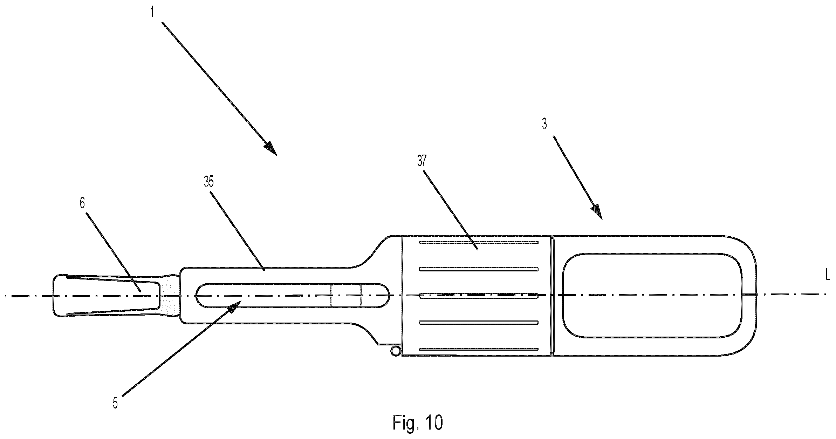

Fig. 10 shows a second example embodiment of a drug delivery device 1 of the present disclosure in form of a semi-reusable autoinjector. Autoinjector 1 comprises a reusable drive unit 3 and a syringe 5 insertable into drive unit 3. The drive unit 3 comprises a distal housing part 35 and a proximal housing part 37 that are connected to each other with a hinge 39. The syringe 5 is insertable into the distal housing part 35 through a proximal opening of the distal housing part 35 by a linear movement in longitudinal direction along longitudinal axis L defined by the drive unit 3. In the second embodiment, the syringe is a safety syringe with an integrated needle shield that covers the needle after the injection is completed. However, the drug delivery device 1 of fig. 10 could also be used for other kinds of safety syringes or for normal prefilled syringes. The drive unit 3 of this example embodiment has a length of 187.9 mm in the longitudinal direction and a circular cross section with a maximum diameter of 30 mm.

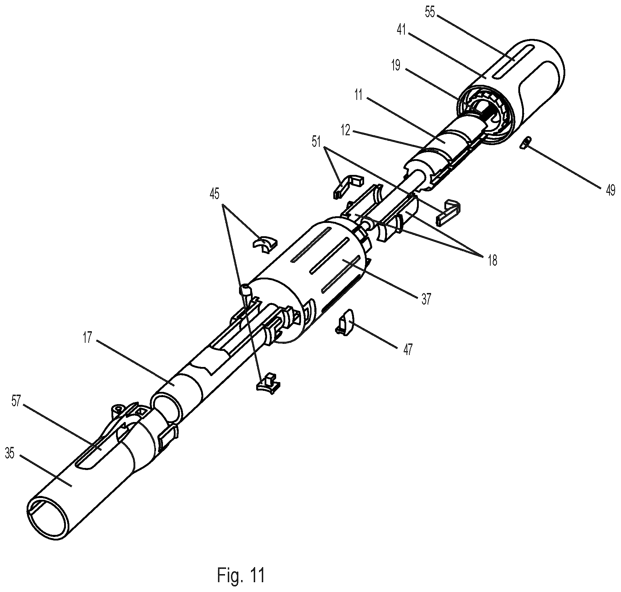

Fig. 11 shows the drive unit 3 of the autoinjector 1 shown in fig. 10 in an exploded view. The main components of the drive unit 3 are a drive unit housing 7 comprising the distal housing part 35 and the proximal housing part 37; a plunger 11 with a lead screw 12; a mechanical drive 19 in form of a compression spring; and a turning knob 41 that is part of the proximal housing part 37. Drive unit 3 also comprises a trigger element 17 and trigger links 18 to trigger the injection when the autoinjector 1 is pressed against the skin at a desired injection site, a pawl 49 that is received in the lead screw 12 of the plunger 11, and sliders 51 that are guided in the lead screw 12 of the plunger 11.

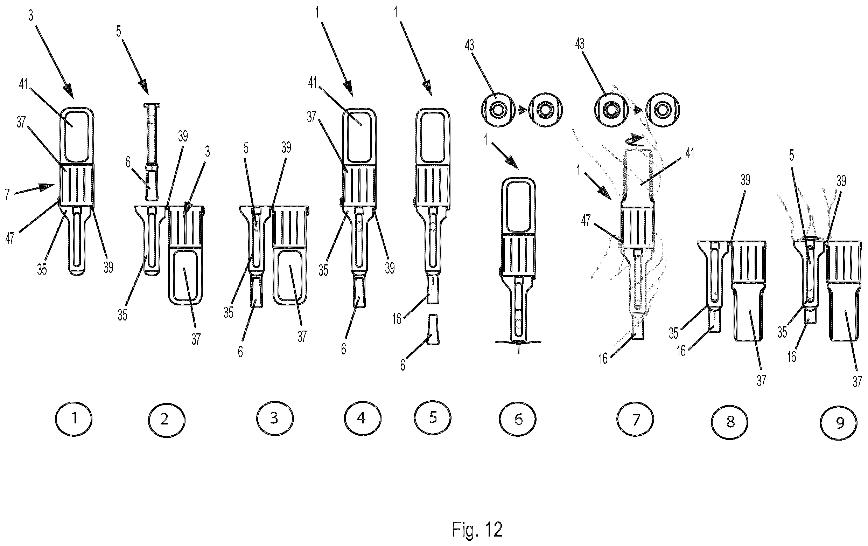

Fig. 12 is a schematic presentation of the loading and injection process of the drug delivery device 1 of fig. 10 and 11.

In a first step, the drive unit 3 respectively the drive unit housing 7 is closed and empty, hence the drive unit 3 does not contain a syringe. The mechanical drive (not shown in fig. 12) is preferably already loaded, which will be explained below. In a second step, the drive unit 3 is opened by bending the drive unit housing 7 around the hinge 39 with other words by moving the housing parts 35, 37 around the hinge 39. To make the opening of the drive unit housing 7 easier, the drive unit 3 can comprise an opening button 47 opposite the hinge 39, that the user can press to open the drive unit housing 7. To make the use of the autoinjector 1 safer and more comfortable, the drive unit 3 can also comprise a detent (not shown in fig. 12) that blocks the hinge 39 in the open position to hold the drive unit housing 7 in the open position.

In a third step the syringe 5 is inserted into the distal housing part 35 of the drive unit housing 7 from a proximal end of the distal housing part 35 in longitudinal direction. In a fourth step, the drive unit housing 7 is closed by bending it back around the hinge 39. In a fifth step, the user pulls off the needle shield 6 of the syringe 5, so that the autoinjector 1 is ready for an injection. To make the needle shield removal easier, the needle shield 6 can comprise a grip area that enables the user to exert a higher force on the needle shield 6.

In a sixth step, the user presses the autoinjector 1 against the skin at a desired injection site with the integrated needle cover 16 of the syringe 5, which is the most distal part of the autoinjector 1, until the injection is complete, or until the user wants to end the injection due to other reasons. The skin contact retracts the integrated needle cover 16 and the trigger element 17 into the drive unit housing 7 as shown in fig. 12 and releases the plunger (not shown in fig. 3). The plunger then pushes a stopper of the syringe 5 in the distal direction to dispense the medicament from the syringe 5. After the injection is complete, in a seventh step, the autoinjector 1 is lifted from the skin, which releases the integrated needle cover 16 to move back to its distal position to cover the needle of the syringe 5 and prevent the user from accidentally touching the needle during the following user steps. After removing the autoinjector from the skin, the user can rotate the turning knob 41 around the longitudinal axis L to load the mechanical drive (not shown in fig. 12). In other embodiments, the user could also rotate the turning knob 41 before the first step or after the fourth step to load the mechanical drive.

As shown in fig. 12, the autoinjector 1 comprises an indicator 43 that shows whether the mechanical drive is in a loaded position or a relaxed position. In the example embodiment of fig. 12, the indicator 43 is in the form of a circle, that goes from empty to full, when the mechanical drive relaxes and goes back from full to empty, when the mechanical drive is loaded. Hence in this embodiment, the indicator 43 shows the amount of medicament that is injected while the plunger moves in the distal direction and allows the mechanical drive to relax. Preferably, the indicator is placed on a proximal end surface of the turning knob 41 so that it can be best seen by a user using the autoinjector 1. Referring to fig. 11, the proximal housing part 37 respectively the turning knob 41 can additionally or alternatively comprise a viewing window 55 that allows the user to see the position of the plunger 11 and hence also gives and indication on how much medicament was dispensed. Additionally or alternatively, the distal housing part 35 can also comprise a viewing window 57 that allows the user to see the position of the stopper of the syringe 5 and hence gives another indication on how much medicament was dispensed.

In an eight step, the user opens the drive unit 3 again by bending the drive unit housing 7 around the hinge 39, preferably by pressing the opening button 47, so that the empty syringe 5 can be removed in a last user step. To remove the syringe 5, the user can for example grab the flange of the syringe 5 to pull the syringe 5 out of the distal housing part 35. Alternatively, the drive unit 3 can comprise a syringe eject button 45 (shown in fig. 10) that pushes the syringe 5 out of the distal housing part 35 so that it is easier for the user to grab and remove the syringe 5. The user can push the syringe eject button 45 in the proximal direction to move the syringe 5 out of the distal housing part 35.

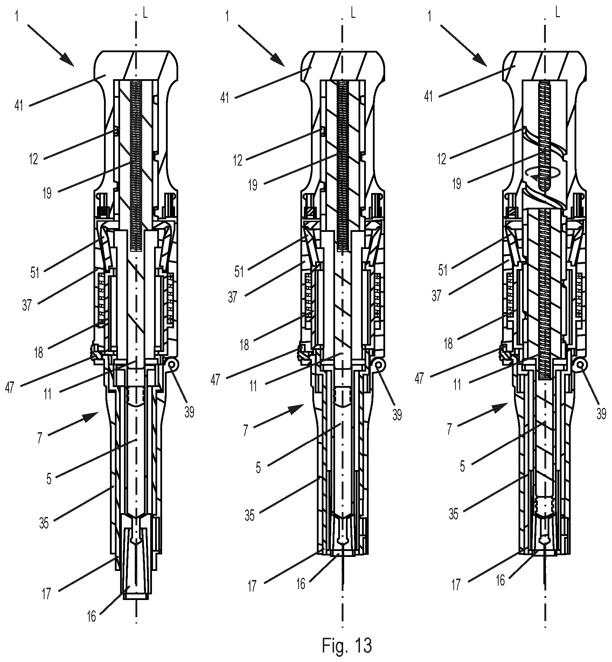

Fig. 13 shows the autoinjector 1 of fig. 10 to 12 before and during the injection, wherein on the left the autoinjector 1 is shown before the injection, in the middle the autoinjector 1 is shown at the start of the injection and on the right the autoinjector 1 is shown in the middle of the injection. Before the injection, the integrated needle cover 16 of the syringe 5 extends from the distal end of the distal housing part 35. The trigger element 17 also extends from the distal end of the distal housing part 35. In this embodiment, the needle cover 16 extends further than the trigger element 17. The plunger 11 is in its most proximal direction and hence the mechanical drive 19 is loaded. The plunger 11 defines an interior adapted to receive the mechanical drive 19 therein and forms a proximal end surface adapted to serve as contact surface for the distal end of the mechanical drive 19. The plunger 11 is held in this position by the sliders 51 engaging in the lead screw 12 of the plunger 11 so that the plunger 11 is prevented from rotating.

As shown in the middle and on the right of fig. 13, when the autoinjector 1 is pressed against the skin, the integrated needle shield 16 of the syringe 5 and the trigger element 17 are pushed in the proximal direction and are retracted into the drive unit housing 7. The trigger element 17 thereby comes into abutment with the trigger link and pushes the trigger link 18 in the proximal direction. This moves the siders 51 out of the engagement with the lead screw 12 of the plunger 11 so that the plunger 11 is now free to rotate under the bias of the loaded mechanical drive 19 to dispense the drug from the syringe 5. In fig. 13 in the middle, the plunger 11 is still in its most proximal position, hence no medicament has been dispensed at this point. On the right of fig. 13, the plunger 11 has moved in the distal direction and is therefore in a more distal position than in the middle of fig. 13. The slider 51 can be made of flexible material, comprise a linkage or comprise a ramp to be able to translate the proximal movement of the trigger link 18 into a radial movement of the slider 51 to bring the slider 51 out of engagement with the lead screw 12 of the plunger 11. The trigger element 17 additionally blocks the opening button 47, when the trigger element 17 is in the retracted position, so that the user cannot accidentally open the autoinjector 1 during the injection.

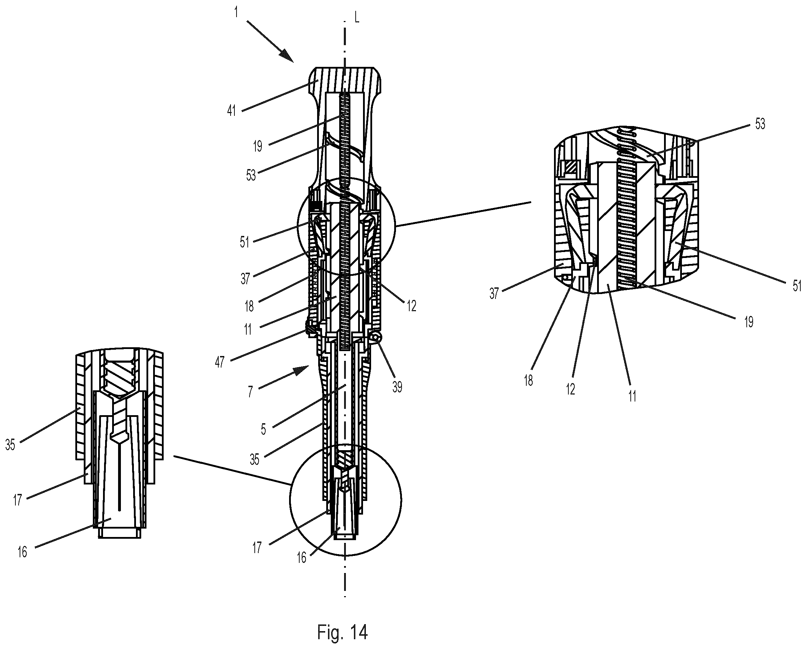

Fig. 14 shows the autoinjector 1 of fig. 10 to 13 after the injection is completed. The integrated needle shield 16 of the safety syringe 5 is then back in its distal position and is additionally locked in this position, so that the needle is securely covered, and the user cannot accidentally touch the needle.

The trigger element 17 also returns to its distal position and the sliders 51 reengage the lead screw 12 of the plunger 11. In addition, the trigger element 17 no longer blocks the opening button 47 so that the user can push the opening button 47 to open the autoinjector 1. In this position, the user can rotate the turning knob 41 move the plunger 11 back in the proximal direction and to load the mechanical drive 19. For this purpose, the proximal housing part 37 has a thread 53 on its inner surface that cooperates with the lead screw 12 on the plunger 11 to translate the rotational movement of the turning knob 41 into a proximal movement of the plunger 11. The turning knob 41 can additionally comprise a ratchet feature, that prevents rotation of the plunger 11 in a first direction that leads to the plunger 11 moving back in the distal direction and only allows rotation of the plunger 11 in the other direction that leads to a movement of the plunger 11 in the proximal direction to load the mechanical drive 19.

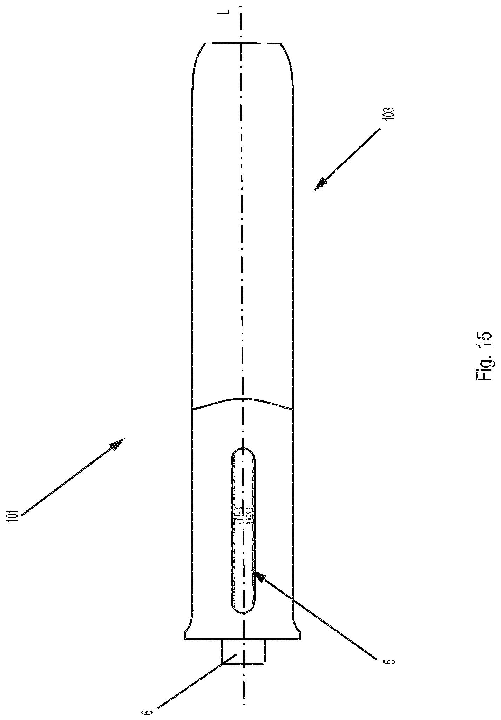

Fig. 15 to 19 show a third example embodiment of a drug delivery device 101 in form of a semi-reusable autoinjector that comprises several features that can be used in a drug delivery device 1 according to the invention that are indicated with the same reference signs used before.

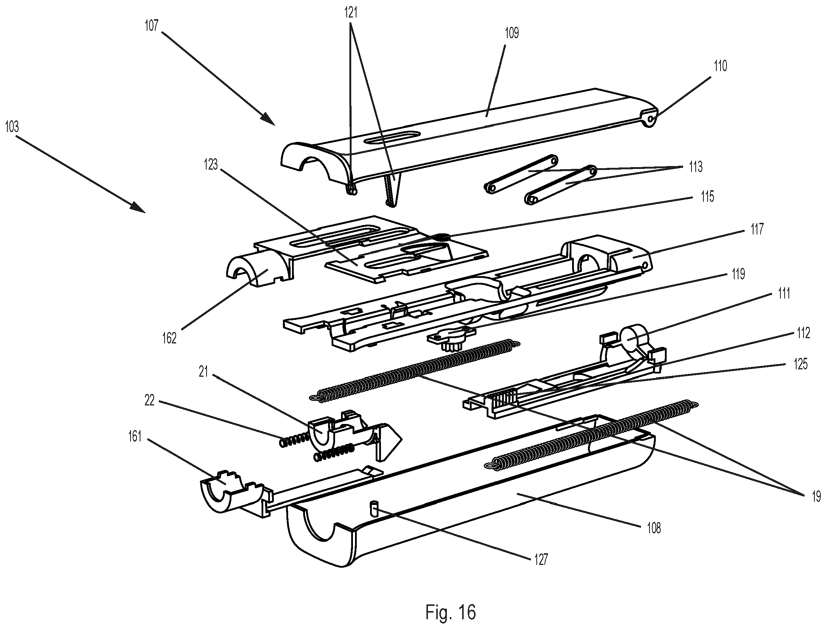

The autoinjector 1 also comprises a reusable drive unit 103 and a disposable syringe 5 insertable into the drive unit 103. The syringe 5 is laterally insertable into the drive unit 103 but differs from the autoinjector 1 of fig. 1 to 9 in the design of the drive unit 103, which will be explained with reference to fig. 16. In this embodiment, the syringe 5 is a safety syringe with a needle retraction mechanism that retracts the needle into the syringe at the end of the injection. However, the drug delivery device 1 of fig. 15 could also be used for other kinds of safety syringes or for normal prefilled syringes. The drive unit 103 of the third example embodiment has length of 170.4 mm in the longitudinal direction and a rectangular cross section with rounded edges and outer dimensions of 34 mm and 25 mm.

Fig. 16 shows the drive unit 103 of the autoinjector 101 in an exploded view. The main components of the drive unit 103 are a drive unit housing 107 comprising a base part 108 and a clamshell door 109 that is connected to the base part 108 at the proximal end of the drive unit housing 107 with a hinge 110, so that the clamshell door 108 can be hinged open to open the drive unit 103 so that the syringe 5 can be inserted; a plunger 111 and a mechanical drive 19 in form of two parallel extension springs. Drive unit 103 also comprises a needle cover 15, linkage elements 113 to translate an opening movement of the clamshell door 109 into a proximal movement of the plunger 111; a torsion spring 115 to bias the needle cover 15 in the distal direction; a syringe holder 117 arranged inside the drive unit housing 7 and having a recess in which the syringe 5 is held when the syringe 5 is inserted; a rotary damper 119 to slow down the movement of the plunger 111 during an injection and a pusher element 21 to remove a needle shield 6 of the syringe 5 (see fig. 15). The rotary damper 119 cooperates with a rack 125 on the distal end of the plunger 111, so that the plunger 111 is slowed down at the beginning of the injection when the force of the mechanical drive 19 is the highest and especially before the plunger 111 contacts the stopper of the syringe 5. Instead of the fixed rack 125 on the plunger 111, in other embodiments a sliding rack that can disengage with the damper 119 during the plunger reset for loading the mechanical drive could be used.

The extensions springs are with one end connected to the drive unit housing 107 that for this purpose comprises two pins 127 near the distal end of the drive unit housing 107, one of which is visible in fig. 16, and the second end is connected to the plunger 111 that for this purpose has two pins 112, one of which is visible in fig. 16. Having two parallel extensions springs makes it possible to place the springs next to the plunger 111 instead of proximal from the plunger 111, which makes the autoinjector 1 shorter in the longitudinal direction. Two extensions springs or two compression springs parallel to the plunger can also be used in other embodiments of inventive drug delivery devices, particularly in the first and second embodiment of autoinjectors previously described herein.

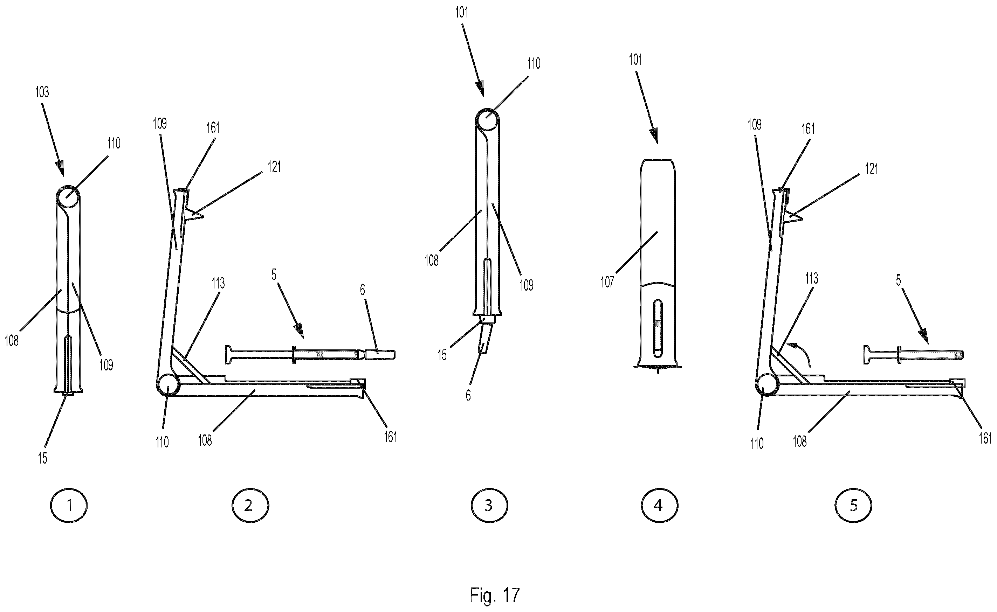

Fig. 17 is a schematic presentation of a loading and injection process of the autoinjector 101 of fig. 15 and 16.

**In** a first step, the drive unit 103 is closed and empty, hence the drive unit 103 does not contain a syringe. **In** the second step, the drive unit 103 is opened by opening the clamshell door 109 and the syringe 5 is inserted into the drive unit 103. The syringe 5 can be fixed in the syringe holder (not shown in fig. 17) by simple clips. To make the syringe easier, the drive unit 103 can comprise a detent that holds the drive unit 103 open while the user inserts the syringe 5. The mechanical drive (not shown in fig. 17) can already be loaded when the drive unit 103 is closed in the first step due to a previous opening of the clamshell door 109, for example to remove a previously used syringe, or it can be loaded by opening the clamshell door 109 in the second step. **In** both cases the mechanical drive is fully loaded after the second step so that the syringe 5 can easily be inserted into drive unit 103. To insert syringe 5 into drive unit 103, the needle shield 15 comprises a lower part 161 connected to the base part 108 and an upper part 162 connected to the clamshell door 162. When the syringe is inserted, it can easily be arranged in the lower part 161 of the needle shield 15.

**In** a third step, the autoinjector 101 is closed by closing the clamshell door 109. This closing movement of the clamshell door 109 removes the needle shield 6 from syringe 5 due to the pusher element 21 (not shown in fig. 17, see fig. 16) that has a recess in which the needle shield 6 is inserted when the syringe 5 is inserted. Projections 121 on the distal end of the clamshell door 109 push the pusher element 21 in the distal direction thereby removing the needle shield 6, when the clamshell door 109 is closed. Due to compression springs 22 (see fig. 16) that bias the pusher element in the proximal direction against the projections 121 of the clamshell door 109, the pusher element 21 also provides a locking feature that holds the clamshell door 109 in the closed position. When the clamshell door 109 is closed, the lower part 161 and the upper part 162 of the needle cover 15 interlock so that they move together in the following steps.

In a fourth step, the user presses the autoinjector 101 against the skin at a desired injection site with the needle cover 15, which is the most distal part of the autoinjector 101, until the injection is complete, or until the user wants to end the injection due to other reasons. The skin contact retracts the needle cover 15 into the drive unit housing 107 as shown in fig. 17 and releases the plunger (not shown in fig. 17). The plunger then pushes a stopper of the syringe 5 in the distal direction to dispense the medicament from the syringe 5.

After the injection is complete, in a fifth step, the autoinjector 101 is lifted from the skin, which releases the needle cover 15 to move back to its distal position under the force of the torsion spring 115 to cover the needle of the syringe 5 and prevent the user from accidentally touching the needle. The user then opens the clamshell door 109 to reveal the empty syringe 5. This opening movement also loads the mechanical drive by the linkage elements 113 that are connected with one end to the clamshell door 109 and the other end connected to the plunger. These linkage elements 113 pull the plunger in the proximal direction when the clamshell door 109 is opened. Instead of the linkage elements, the drive unit could comprise gears to load the mechanical drive, when the clamshell door is opened. The drive unit 103 can further comprise a locking protrusion that locks the plunger in its most proximal position in which the mechanical drive is loaded. Lastly, the user can remove the empty syringe 5 from the drive unit 103, for example by grabbing the shaft of the syringe plunger, by pushing the syringe out of the drive unit from the back or by pressing a release button. After the syringe 5 is removed, the clamshell door 109 can be closed for storage of the autoinjector 101.

Fig. 18 and 19 illustrate the torsion spring 115 that biases the needle cover 15 in the distal direction in detail. One end of the torsion spring 115 is connected to the needle cover 15 and the second end is connected to the drive unit housing 107. **In** this embodiment, the second end is connected to an intermediate plate 123 that is connected to the clamshell door 109 by recesses receiving the projections 121 of the clamshell door 109. However, the second end of the torsion spring 115 could also be connected to the clamshell door 109 itself or to another part of the drive unit housing 107. The torsion spring 115 to bias the needle cover 15 can be used in other inventive drug delivery devices, particularly in the first or second embodiment previously described.

The previous description of the disclosed embodiments is provided to enable a person skilled in the art to make or use the disclosed embodiments. Various modifications to these embodiments will be readily apparent to those skilled in the art, and the principles defined herein may be applied to other embodiments without departing from the scope of the disclosure. Thus, the present disclosure is not intended to be limited to the embodiments shown herein but is to be accorded the widest scope possible consistent with the principles and novel features as previously shown and described.

### LIST OF DESIGNATIONS

- 1: drug delivery device
- 3: drive unit
- 5: syringe
- 6: needle shield
- 7: drive unit housing
- 8: lower housing part
- 9: upper housing part
- 10: syringe holder
- 11: plunger
- 12: lead screw
- 13: puller
- 14: plate-like protrusion
- 15: needle cover
- 16: integrated needle cover
- 17: trigger element
- 18: trigger link
- 19: mechanical drive
- 21: pusher element
- 22: compression spring
- 23: insertion opening
- 25: push button
- 27: lever arrangement
- 29: pivoting lever
- 31: puller projection
- 33: plunger projection
- 35: distal housing part
- 37: proximal housing part
- 39: hinge
- 41: turning knob
- 43: indicator
- 45: syringe eject button
- 47: opening button
- 49: pawl
- 51: slider
- 53: thread
- 55, 57: viewing window
- 101: autoinjector
- 103: drive unit
- 107: drive unit housing
- 108: base part
- 109: clamshell door
- 110: hinge
- 111: plunger
- 112: pin
- 113: linkage element
- 115: torsion spring
- 117: syringe holder
- 119: rotary damper
- 121: projection
- 123: intermediate plate
- 125: rack
- 127: pin
- 151, 152: needle cover half sleeve
- 153: hinge
- 154: entrance
- 155: bottom half
- 156, 157: door portion
- 158, 159: hinge
- 160: opening
- 161: lower part needle cover
- 162: upper part needle cover
- L: longitudinal axis

## Claims

1. A loading mechanism for a drug delivery device (1) for dispensing a liquid drug, the loading mechanism comprising a drive unit (3) and a syringe (5) insertable into the drive unit (3),
the drive unit (3) comprising a drive unit housing (7) defining a longitudinal axis (L), a plunger (11) movable inside the drive unit housing (7) in a dispensing direction to dispense the liquid drug and a mechanical drive (19), preferably a compression spring, arranged inside the drive unit housing (7) for moving, when loaded, the plunger (11) in the dispensing direction,
wherein the drive unit housing (7) comprises an insertion opening (23) to insert and remove the syringe (5),
wherein the drive unit (3) further comprises a puller (13) that is movable in the proximal direction to pull the plunger (11) in the proximal direction to load the mechanical drive (19),
**characterized in that** the puller (13) is adapted to block the insertion opening (23) before moving in the proximal direction and to open the insertion opening (23) by moving in the proximal direction.

2. Loading mechanism according to claim 1, wherein the insertion opening (23) is located laterally on the drive unit housing (7) so that the syringe (7) is laterally insertable.

3. Loading mechanism according to one of the preceding claims, wherein the puller (13) comprises a first projection (31) and the plunger (11) comprises a second projection (33) that abut when the puller (13) is moved in the proximal direction to pull the plunger (11) in the proximal direction.

4. Loading mechanism according to one of the preceding claims, wherein the puller (13) is adapted to remove a needle shield (6) of the syringe (5), when the puller (13) is moved in the distal direction to close the drug delivery device (1).

5. Loading mechanism according to one of claims 1 to 3, wherein the drive unit (3) comprises a push button (25) that is adapted to remove a needle shield (6) of the syringe (5).

6. Loading mechanism according to one of claims 1 to 3, wherein the drive unit (3) comprises a pivoting lever (29) that automatically removes a needle shield (6) of the syringe (5), when the syringe (5) is inserted into the drive unit (3).

7. Loading mechanism according to one of the preceding claims, wherein the drive unit (3) comprises a needle cover (15) that is biased in the distal direction by a torsion spring (115).

8. Loading mechanism according to one of the preceding claims, wherein the syringe (5) is inserted laterally through a needle cover (15) of the drive unit (3), wherein the needle cover (15) is reversibly moved to an open position by the lateral insertion of the syringe (5) to enable the insertion of the syringe (5).

9. Loading mechanism according to claim 8, wherein the needle cover (15) comprises a section movable around a hinge (153, 158, 159), or a section made of a deformable material.

10. Loading mechanism for dispensing a liquid drug, the loading mechanism comprising a drive unit (3) and a syringe (5) insertable into the drive unit (3),
the drive unit (3) comprising a drive unit housing (7) defining a longitudinal axis (L), a plunger (11) movable inside the drive unit housing (7) in a dispensing direction to dispense the liquid drug and a mechanical drive (19), preferably a compression spring, arranged inside the drive unit housing (7) for moving, when loaded, the plunger (11) in the dispensing direction,
wherein the drive unit housing (7) comprises a distal housing part (35) and a proximal housing part (37) connected by a hinge (39) so that the drive unit (3) can be opened by moving the housing parts (35, 37) around the hinge (39),
**characterized in that** the proximal housing part (37) comprises a turning knob (41) to load the mechanical drive (19) by a rotational movement of the turning knob (41) around the longitudinal axis (L).

11. Loading mechanism according to claim 10, wherein the syringe (5) is insertable into the distal housing part (35) in a longitudinal direction from a proximal opening of the distal housing part (35).

12. Loading mechanism according to claim 10 or 11, wherein the plunger (11) comprises a lead screw (12) and the proximal housing part (37) comprises a thread (53) to translate the rotational movement of the turning knob (41) to a proximal movement of the plunger (11).

13. Loading mechanism according to one of claims 10 to 12, wherein the drive unit (3) comprises an opening button (47) to open the drive unit (3) and an injection trigger element (17) movable between an extended position in which the opening button (47) can be pressed and a retracted position in which it releases the plunger (11) and blocks the opening button (47).

14. Loading mechanism according to one of the preceding claims, wherein the syringe (5) is a safety syringe with a needle retraction mechanism or a safety syringe with an integrated needle cover (16).

15. Drug delivery device (1) comprising a loading mechanism according to one of the preceding claims, wherein preferably, the drive unit housing (7), the plunger (11), the puller (13), the needle cover (15), the turning knob (41) and/or the trigger element (17) are injection-molded thermoplastic plastic parts.
